(19) Europäisches Patentamt
European Patent Office
Office européen des brevets

(11) Publication number: **0 004 171**
**B1**

(12) **EUROPEAN PATENT SPECIFICATION**

(21) Application number: 79300324.5

(22) Date of filing: 05.03.79

(51) Int. Cl.³: C 07 D 487/04, A 01 N 43/90//(C07D487/04, 251/00, 231/00)

(54) **Pyrazolotriazinedione derivatives, process for their preparation and herbicidal compositions containing them.**

(30) Priority: 10.03.78 GB 954678

(43) Date of publication of application: 19.09.79 Bulletin 79/19

(45) Publication of the grant of the European patent: 11.02.81 Bulletin 81/6

(84) Designated Contracting States: BE CH DE FR GB IT NL

(56) References cited:
FR - A - 2 351 118

JOURNAL OF HETEROCYCLIC CHEMISTRY, 12 (5), October 1975; Provo Utah, USA

K. SENGA et al. "Synthesis of 1, 3-Dialkylpyrazolo (1, 5-a)-1,3,5-triazine-2,4-diones Isomers of 1, 3-Dialkylxanthines", pages 893—898

(73) Proprietor: IMPERIAL CHEMICAL INDUSTRIES LIMITED
Imperial Chemical House Millbank
London SW1P 3JF (GB)

(72) Inventor: Cartwright, David
1 Stonehaven Drive Woodley
Reading Berkshire (GB)

(72) Inventor: Urlwin-Smith, Philip
Earley Cottage Earleydene
Sunninghill Berkshire (GB)

(72) Inventor: Collins, David John
10 Ardwell Close
Crowthorne Berkshire (GB)

(74) Representative: Downes, John Edward et al,
Imperial Chemical Industries Limited Legal Department: Patents Thames House North Millbank
London SW1P 4QG (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 004 171 B1

Courier Press, Leamington Spa, England.

# Pyrazolotriazinedione derivatives, process for their preparation and herbicidal compositions containing them

The present invention relates to heterocyclic compounds, and in particular to novel pyrazolotriazinediones useful as herbicides. Pyrazolotriazinedione compounds have been described by K. Senga et al, J. Heterocyclic Chem. 1975, volume 12, pages 893—898. Triazolotriazinedione compounds have been described in French Patent Specification no. 2 351 118.

According to the present invention we provide herbicidal pyrazolotriazinedione compounds of formula (I):

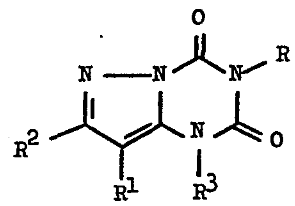

(I)

and tautomers and salts thereof, where R is a cycloalkyl or branched alkyl group of 3 to 10 carbon atoms, $R^1$ is hydrogen, lower alkyl, chlorine, fluorine, bromine, iodine, cyano, nitro, carbamoyl or lower alkanoylamido; $R^2$ is hydrogen or lower alkyl, lower mono- or di-alkylamino, lower alkoxy or lower alkylthio; and $R^3$ is hydrogen or lower alkyl; and salts thereof with bases. Compounds in which $R^1$ is halogen, methyl, or cyano are preferred. By 'lower' (alkyl, etc) we mean 'containing up to 5 carbon atoms'.

In the compounds of the invention, R may be, for example *iso*propyl, 1-methylpropyl, *iso*butyl, *cyclo*pentyl or *cyclo*hexyl. A particular example of a compound according to the invention is the one in which R is *iso*propyl, $R^1$ is chlorine, $R^2$ is hydrogen, and $R^3$ is hydrogen. Further examples of compounds of the invention are listed in Table I.

The compounds of the invention are potentially capable of existing in tautomeric forms. Thus, the compound where R is isopropyl, and $R^1$, $R^2$ and $R^3$ are each hydrogen, could in principle exist in the following form among others:

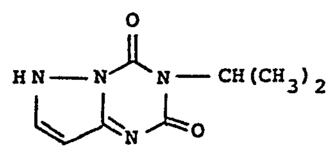

The structural formula (I) given above is intended to be representative of and to embrace all tautomeric forms of the compounds of the invention.

The compounds of the invention form salts with both acids and bases. Acid addition salts include salts formed from mineral acids, for example hydrochloric, hydrobromic, sulphuric, nitric and phosphoric acids. Salts with bases include salts of metals and salts formed from ammonium and substituted ammonium cations. Among the metal salts are those in which the metal cation is an alkali metal cation for example sodium, potassium, or lithium, or an alkaline earth metal cation, for example calcium or magnesium. The substituted ammonium cations include mono-, di-, tri-, and tetra-substituted ammonium cations in which the substituents may be, for example, an alkyl or alkenyl radical of 1 to 20 carbon atoms optionally containing one or more hydroxy or alkoxy substituents.

TABLE I

| Compound Number | R | $R^1$ | $R^2$ | $R^3$ | Melting Point °C |
|---|---|---|---|---|---|
| 1 | *iso*propyl | H | H | H | >260 |
| 2 | *iso*propyl | CN | H | H | 132 |
| 3 | *iso*propyl | H | $CH_3$ | H | 245 |
| 4 | *iso*propyl | CN | $CH_3$ | H | 127 |
| 5 | *iso*propyl | H | $CH_3$ | $CH_3$ | 160 |
| 6 | *iso*propyl | CN | $CH_3S-$ | H | 268 |
| 7 | *iso*propyl | $-CONH_2$ | $CH_3S-$ | H | 260 |
| 8 | *iso*propyl | Br | H | H | 214 |
| 9 | *iso*propyl | CN | $-N(CH_3)_2$ | H | 200 |
| 10 | *iso*propyl | CN | $CH_3O-$ | H | 255 |
| 11 | *iso*propyl | Br | $CH_3-$ | H | 263 |
| 12 | *iso*propyl | H | H | $CH_3$ | 150 |
| 13 | *iso*propyl | $-NO_2$ | H | H | 172 |
| 14 | *iso*propyl | Cl | H | H | 162 |
| 15 | *iso*propyl | I | H | H | 222 |
| 16 | *iso*propyl | H | $CH_3S$ | H | 160 |
| 17 | *cyclo*hexyl | H | H | $CH_3$ | 219 |
| 18 | *cyclo*hexyl | H | $CH_3$ | H | 194 |
| 19 | *cyclo*hexyl | H | $CH_3$ | $CH_3$ | 164 |

TABLE I (Continued)

| Compound Number | R | $R^1$ | $R^2$ | $R^3$ | Melting Point °C |
|---|---|---|---|---|---|
| 20 | *iso*propyl | $CH_3$ | H | H | 221 |
| 21 | *iso*propyl | $CH_3$ | H | $CH_3$ | 104 |
| 22 | 1-methylpropyl | H | H | H | 250 |
| 23 | 1-methylpropyl | H | H | $CH_3$ | 104 |
| 24 | 1-methylpropyl | Br | H | H | 152 |
| 25 | 1-methylpropyl | Br | H | $CH_3$ | 100 |
| 26 | *iso*propyl | $-CONH_2$ | $CH_3$ | H | 300 |
| 27 | *cyclo*hexyl | H | H | H | >270 |
| 28 | *cyclo*hexyl | Br | H | H | 214 |
| 29 | *cyclo*hexyl | Br | H | $CH_3$ | 170 |
| 30 | *cyclo*hexyl | Cl | H | H | 210 |
| 31 | *iso*propyl | Cl | $CH_3$ | H | 225 |
| 32 | *iso*propyl | I | $CH_3$ | H | 240 |
| 33 | *iso*propyl | $NO_2$ | $CH_3$ | H | 170 |
| 34 | *iso*propyl | Br | $CH_3$ | $CH_3$ | 168 |
| 35 | *cyclo*hexyl | $NO_2$ | H | H | 228 |
| 36 | *iso*propyl | Br | H | $CH_3$ | 153 |
| 37 | *iso*propyl | $CH_3CONH-$ | H | H | 225 |
| 38 | *cyclo*hexyl | H | $CH_3$ | H | 194 |

TABLE I (Continued)

| Compound Number | R | $R^1$ | $R^2$ | $R^3$ | Melting Point °C |
|---|---|---|---|---|---|
| 39 | *cyclo*hexyl | Br | $CH_3$ | H | 237 |
| 40 | *cyclo*hexyl | $NO_2$ | $CH_3$ | H | 200 |
| 41 | *cyclo*hexyl | H | $CH_3$ | $CH_3$ | 171 |
| 42 | *cyclo*hexyl | Br | *iso*propyl | H | 230 |
| 43 | *iso*butyl | H | H | H | 270 |
| 44 | *cyclo*pentyl | H | H | H | >270 |
| 45 | *iso*butyl | Br | H | H | 247 |
| 46 | *iso*butyl | H | H | $CH_3$ | 117 |
| 47 | *cyclo*pentyl | Br | H | H | 208 |
| 48 | *cyclo*pentyl | H | H | $CH_3$ | 153 |
| 49 | *cyclo*hexyl | $CH_3$ | H | H | 237 |
| 50 | *cyclo*hexyl | $CH_3$ | H | $CH_3$ | 164 |
| 51 | *cyclo*pentyl | Br | H | $CH_3$ | 140 |
| 52 | *iso*propyl | $CH_3$ | $CH_3$ | H | 168 |
| 53 | *cyclo*hexyl | $CH_3$ | $CH_3$ | H | 180 |
| 54 | *iso*propyl | $CH_3$ | $CH_3$ | $CH_3$ | 149 |
| 55 | *cyclo*hexyl | $CH_3$ | $CH_3$ | $CH_3$ | 190 |
| 56 | 1-methylpropyl | H | $CH_3$ | H | 172 |
| 57 | 1-methylpropyl | Br | $CH_3$ | H | 195 |

TABLE I (Continued)

| Compound Number | R | $R^1$ | $R^2$ | $R^3$ | Melting Point °C |
|---|---|---|---|---|---|
| 58 | 1-methylpropyl | H | $CH_3$ | $CH_3$ | 85 |
| 59 | *iso*butyl | H | $CH_3$ | H | 256 |
| 60 | 1-methylpropyl | Br | $CH_3$ | $CH_3$ | 129 |
| 61 | *cyclo*pentyl | H | $CH_3$ | H | 242 |
| 62 | *iso*butyl | Br | $CH_3$ | H | 240 (s) |
| 63 | *iso*butyl | H | $CH_3$ | $CH_3$ | 178 (s) |
| 64 | *iso*butyl | Br | $CH_3$ | $CH_3$ | 106 |
| 65 | *cyclo*pentyl | Br | $CH_3$ | H | 251 |
| 66 | *cyclo*pentyl | H | $CH_3$ | $CH_3$ | 141 |
| 67 | *cyclo*pentyl | Br | $CH_3$ | $CH_3$ | 159 |
| 68 | 1-methylpropyl | $CH_3$ | H | H | 132 |
| 69 | *iso*butyl | $CH_3$ | H | H | 205 |
| 70 | 1-methylpropyl | $CH_3$ | H | $CH_3$ | 97 |
| 71 | *cyclo*hexyl | H | H | $C_2H_5$ | 140 |
| 72 | *cyclo*hexyl | H | H | $C_3H_7$ | 129 |
| 73 | *cyclo*hexyl | I | H | H | 226 |

NOTES

With regard to the melting points in the above Table, the symbol "(s)" means that the compound sublimed. The melting point given for compound 11 is for the hydrobromide of this compound.

The invention further comprises a herbicidal composition comprising as an active ingredient a compound of formula (I) in admixture with a carrier comprising a solid or liquid diluent; and a method of inhibiting the growth of unwanted plants which comprises applying to the plants or the locus thereof a compound of the formula (I).

Compounds according to the invention may be active against monocotyledons and dioctyledons, both pre- and post-emergence, but are usually more active against dicotyledons, particularly post-emergence. Certain of the compounds have potentially valuable selective herbicidal properties, e.g. Compounds nos 8 and 14 of Table I are expected to have utility as pre-emergence herbicides in maize.

Some of the compounds also show fungicidal activity.

Compounds according to the invention may be prepared by condensation of 3-amino pyrazoles with alkyl or cycloalkyl bis-chloroformylamines according to the general reaction scheme A shown below.

Scheme A

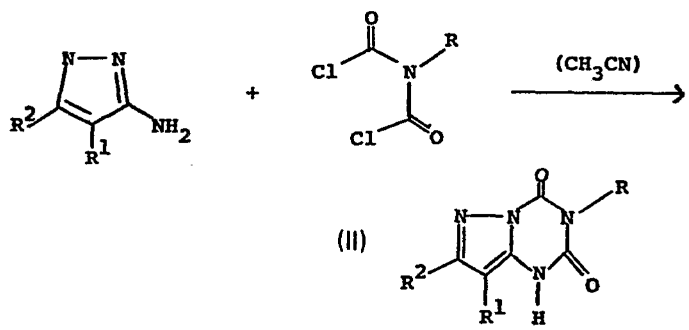


(II)

The product so obtained may if required be reacted with a base and a lower alkyl halide to form compounds according to the invention in which $R^3$ is lower alkyl. Compounds in which $R^1$ is bromine, chlorine, iodine or nitro may also be obtained by reacting compounds of formula I wherein $R^1$ is hydrogen with a halogenating or nitrating agent. The 3-aminopyrazoles used as starting materials in Scheme A are in general known; where not previously described, they may be prepared by application of standard conventional methods.

In using the compounds as herbicides, the amount of the compound to be applied will depend upon a number of factors, for example the particular plant species whose growth is to be inhibited, but in general an amount of from 0.5 to 10 kilograms per hectare is usually suitable. The skilled worker in the art will readily be able to determine suitable amounts for use by means of standardised routine tests, without undue experimentation.

The compounds of the invention are preferably applied in the form of compositions, in which the active ingredient is mixed with a carrier comprising a solid or liquid diluent. Preferably the composition further comprises a surface-active agent.

The solid compositions of the invention may be for example, in the form of dusting powders, or may take the form of granules. Suitable solid diluents include, for example, kaolin, bentonite, kieselguhr, dolomite, calcium carbonate, talc, powdered magnesia, and Fuller's earth.

Solid compositions may also be in the form of dispersible powders or grains comprising in addition to the active ingredient, a wetting agent to facilitate the dispersion of the powder or grains in liquids. Such powders or grains may include fillers, suspending agents and the like.

Liquid compositions include aqueous solutions, dispersions and emulsions containing the active ingredient preferably in the presence of one or more surface active agents. Water or organic liquids may be used to prepare solutions, dispersions, or emulsions or the active ingredient. The liquid compositions of the invention may also contain one or more corrosion inhibitors for example lauryl isoquinolinium bromide.

Surface-active agents may be of the cationic, anionic or non-ionic type. Suitable agents of the cationic type include for example quaternary ammonium compounds, for example cetyltrimethyl ammonium bromide. Suitable agents of the anionic type include for example soaps, salts of aliphatic mono-esters of sulphuric acid, for example sodium lauryl sulphate; and salts of sulphonated aromatic compounds, for example sodium dodecylbenzenesulphonate, sodium, calcium and ammonium lignosulphonate, sodium butylnaphthalene sulphonate, and a mixture of the sodium salts of diisopropyl- and triisopropyl-naphthalenesulphonic acid. Suitable agents of the non-ionic type include, for example, the condensation products of ethylene oxide with fatty alcohols such as oleyl alcohol and cetyl alcohol, or with alkyl phenols such as octyl-phenol, nonylphenol, and octylcresol. Other non-ionic agents are the

partial esters derived from long chain fatty acids and hexitol anhydrides, for example sorbitol monolaurate; the condensation products of the said partial esters with ethylene oxide and the lecithins.

The compositions which are to be used in the form of aqueous solutions, dispersions or emulsions are generally supplied in the form of a concentrate containing a high proportion of the active ingredient, the concentrate being diluted with water before use. These concentrates are usually required to withstand storage for prolonged periods and after such storage to be capable of dilution with water in order to form aqueous preparations which remain homogeneous for a sufficient time to enable them to be applied by conventional spray equipment. In general concentrates may conveniently contain from 10 to 85% and preferably from 25 to 60% by weight of active ingredient. Dilute preparations ready for use may contain varying amounts of the active ingredient, depending upon the purpose for which they are to be used; however, dilute preparations suitable for many uses contain between 0.01% and 10% and preferably between 0.1% and 1% by weight of the active ingredient.

The compounds of the invention may be used in admixture with other herbicides. Accordingly the invention further provides a herbicidal composition comprising a mixture of at least one herbicide of formula (I) above with at least one other herbicide.

The other herbicide may be any herbicide not having the formula (I). It may be a herbicide having a similar spectrum of herbicidal effect, or it may be a herbicide having a complementary action.

Examples of herbicides which may be mixed with the compounds of the invention include the following:—

A. Bipyridylium herbicides, for example paraquat dichloride (1,1′-dimethyl-4,4′-bipyridylium dichloride) and diquat dibromide (1,1′-ethylene-2,2′-bipyridylium dibromide).

B. Glyphosate (N-phosphonomethylglycine) and its salts and esters.

C. Bentazon (3-isopropyl-(1H)-benzo-2,1,3-thiazine-4-one 2,2-dioxide).

D. Hormone herbicides
e.g. MCPA (4-chloro-2-methylphenoxyacetic acid) 2,4-D (2,4-dichlorophenoxyacetic acid)
Dichlorprop (2-[2,4-dichlorophenoxy]propionic acid)
2,4,5-T (2,4,5-trichlorophenoxyacetic acid)
Mecoprop (2-[4-chloro-2-methylphenoxy]propionic acid).

E. Urea herbicides
e.g. Chloroxuron (3-[4-(4-chlorophenoxy)phenyl]-1,1-dimethyl urea)
Diuron (1-[3,4-dichlorophenyl]-3,3-dimethyl urea)
Fluometuron (1-[metatrifluoromethylphenyl]-3,3-dimethyl urea).

F. Triazine herbicides
e.g. simazine (2-chloro-4,6-diethylamino-1,3,5-triazine)
atrazine (2-chloro-4-ethylamino-6-isopropylamino-1,3,5-triazine)

G. 1-alkoxy-1-alkyl-3-phenylurea herbicides
e.g. linuron (3-[3,4-dichlorophenyl]-1-methoxy-1-methyl urea)
monolinuron (3-[4-chlorophenyl]-1-methoxy-1-methyl urea).

H. 1,2,4-Triazine-5-one herbicides
e.g. metamitron (4-amino-4,5-dihydro-3-methyl-6-phenyl-1,2,4-triazin-5-one)
metribuzin (4-amino-6-tertbutyl-4,5-dihydro-3-methylthio-1,2,4-triazin-5-one).

I. Anilide herbicides
e.g. butachlor (N-butoxymethyl-$\alpha$-chloro-2′,6′-diethylacetanilide)
alachlor (N-methoxymethyl-$\alpha$-chloro-2′,6′-diethylacetanilide)
and propanil (3,5-dichloropropionanilide).

J. Haloalkanoic acids
e.g. dalapon (2,2-dichloropropionic acid)
TCA (trichloroacetic acid)

K. Diphenyl ether herbicides
e.g. fluorodifen (4-nitrophenyl 2′-nitro-4′-trifluoromethylphenyl ether)
2-nitro-5-(2-chloro-4-trifluoromethylphenoxy)benzoic acid
and 2-chlorophenyl-3′-ethoxy-4′-nitro-4-trifluoromethylphenyl ether

The mixtures of the invention generally contain from 0.1 to 10 parts, conveniently from 0.2 to 2 parts by weight of herbicide of formula (I) per part by weight of the other herbicide, depending upon the relative activity of the components. The amount of the mixture to be applied will depend upon a number of factors, for example the particular plant species to which the mixture is to be applied, but in general an amount of from 0.1 to 5.0 kilograms per hectare will usually be suitable.

The invention is illustrated by the following Examples, in which all parts are by weight and all temperatures in degrees Centigrade unless otherwise specified.

## EXAMPLE 1

This Example illustrates the preparation of 3-*iso*-propylpyrazolo (1,5-a)-1,3,5-triazine-2,4-dione (Compound No 1 of Table I).

3-Amino pyrazole (8.3 g) in a small volume of acetonitrile was added dropwise with stirring to a solution of isopropyl bis-chloroformylamine (19.0 g) in acetonitrile (150 ml). After complete addition

the resulting mixture was refluxed for 4 hours, until no more hydrochloric acid gas was evolved. The resulting mixture was cooled to 0°C and filtered to yield a crude product (10 g). The structure of the compound was confirmed by nuclear magnetic resonance spectroscopy (NMR). M.p. above 260°C.

EXAMPLE 2

This Example illustrates the preparation of 8-bromo-3-*iso*propylpyrazolo-(1,5-a)-1,3,5-triazine-2,4-dione (Compound No 8 of Table I).

Bromine (8.1 g) in carbon tetrachloride (25 ml) was added dropwise with stirring at room temperature to a suspension/solution of the product of Example 1 (9.7 g) in glacial acetic acid (75 ml). After complete addition the resulting mixture was stirred overnight at room temperature and then diluted with 75 mls $CCl_4$. The resulting mixture was filtered and the solid dried and recrystallised from toluene/petrol. Yield 10.2 g; M.p. 226°C. The structure was confirmed by NMR.

EXAMPLE 3

This Example illustrates the herbicidal properties of the compounds used in the process of the invention. Each compound (0.12 g) was formulated for test by mixing it with 5 ml of an emulsion prepared by diluting 100 ml of a solution containing 21.8 grams per litre of Span 80 and 78.2 grams per litre of Tween 20 in methyl cyclohexanone to 500 ml with water. Span 80 is a Trade Mark for a surface-active agent comprising sorbitan monolaurate. Tween 20 is a Trade Mark for a surface-active agent comprising a condensate of twenty molar proportions of ethylene oxide with sorbitan monooleate. The mixture of the compound and the emulsion was shaken with glass beads and diluted to 12 ml with water.

The spray composition so prepared was sprayed on to young pot plants (post-emergence test) of the species named in Table II below, at a rate equivalent to 1000 litres per hectare (10 kilograms of test compound per hectare). Damage to plants was assessed 14 days after spraying by comparison with untreated plants, on a scale of 0 to 3 where 0 is no effect and 3 represents 75 to 100% kill. In a test for pre-emergence herbicidal activity, seeds of the test species were placed on the surface of fibre trays of soil and were sprayed with the compositions at the rate of 1000 litres per hectare. The seeds were then covered with further soil. Three weeks after spraying, the seedlings in the sprayed fibre trays were compared with the seedlings in unsprayed control trays, the damage being assessed on the same scale of 0 to 3. The results are given in Table II below:—

TABLE II

| Compound No. | Rate of Application kg/ha | Pre- or Post-Emergence Application | Test Plants | | | | | |
|---|---|---|---|---|---|---|---|---|
| | | | Lt | To | Ot/Av | Ll | Cn | St |
| 1 | 10 | Pre | 3 | 2 | 3 | 2 | 0 | – |
| | | Post | 2 | 3 | 1 | 3 | 2 | 0 |
| 2 | 10 | Pre | – | 0 | 0 | 0 | 0 | 0 |
| | | Post | 3 | 3 | 1 | 0 | 0 | 1 |
| 3 | 10 | Pre | 0 | 3 | 3 | 2 | 0 | 3 |
| | | Post | 3 | 3 | 3 | 3 | 1 | – |
| 4 | 10 | Pre | 0 | 0 | 0 | 0 | 0 | 0 |
| | | Post | 2 | 2 | 0 | 1 | 0 | 3 |
| 5 | 10 | Pre | 3 | 3 | 3 | 3 | 0 | 3 |
| | | Post | 2 | 2 | 1 | 0 | 0 | 2 |
| 6 | 10 | Pre | 0 | 0 | 0 | 0 | 0 | 0 |
| | | Post | 3 | 0 | 0 | 0 | 0 | 0 |
| 7 | 10 | Pre | – | 0 | 0 | 0 | 0 | 0 |
| | | Post | 3 | 0 | 0 | 0 | 0 | 0 |

TABLE II (Continued)

| Compound No. | Rate of Application kg/ha | Pre or Post Emergence Application | Test Plants Lt | To | Ot/Av | Ll | Cn | St |
|---|---|---|---|---|---|---|---|---|
| 8 | 10 | Pre | 3 | 3 | 3 | 2 | 0 | 3 |
| | | Post | 3 | 3 | 2 | 2 | 0 | 2 |
| 9 | 10 | Pre | — | 0 | 0 | 0 | 0 | 0 |
| | | Post | 3 | 1 | 1 | 0 | 0 | 0 |
| 10 | 10 | Pre | 3 | 3 | 3 | 2 | 0 | 3 |
| | | Post | 3 | 3 | 0 | 0 | 0 | 3 |
| 11 | 10 | Pre | 3 | 3 | 3 | 3 | 0 | 3 |
| | | Post | 3 | 2 | 0 | 0 | 0 | 0 |
| 12 | 10 | Pre | 3 | 3 | 3 | 2 | 0 | 3 |
| | | Post | 1 | 2 | 0 | 0 | 0 | 3 |
| 13 | 10 | Pre | 1 | 3 | 3 | 0 | 0 | 1 |
| | | Post | 3 | 3 | 3 | 0 | 2 | 3 |
| 14 | 10 | Pre | 3 | 3 | 3 | 0 | 0 | 2 |
| | | Post | 3 | 3 | 3 | 3 | 1 | 3 |

TABLE II (Continued)

| Compound No. | Rate of Application kg/ha | Pre- or Post-Emergence Application | Test Plants | | | | | |
|---|---|---|---|---|---|---|---|---|
| | | | Lt | To | Ot/Av | Ll | Cn | St |
| 15 | 10 | Pre | 3 | 3 | 3 | 2 | 2 | 3 |
| | | Post | 3 | 3 | 3 | 2 | 1 | 3 |
| 16 | 10 | Pre | – | 3 | 3 | 1 | 0 | 3 |
| | | Post | 1 | 2 | 1 | 0 | 1 | 3 |
| 20 | 10 | Pre | 3 | 3 | 3 | 3 | 0 | 3 |
| | | Post | 3 | 3 | 3 | 3 | 3 | 3 |
| 21 | 10 | Pre | 3 | 3 | 2 | 2 | 0 | 3 |
| | | Post | 3 | 3 | 2 | 0 | – | 3 |
| 22 | 10 | Pre | 3 | 3 | 3 | 3 | 0 | 3 |
| | | Post | 3 | 3 | 3 | 3 | 0 | 3 |
| 23 | 10 | Pre | 3 | 3 | 3 | 3 | 0 | 3 |
| | | Post | 3 | 3 | 3 | 3 | 0 | 3 |
| 24 | 10 | Pre | 3 | 3 | 3 | 3 | 0 | 3 |
| | | Post | 3 | 3 | 3 | 3 | 2 | 3 |

TABLE II (Continued)

| Compound No. | Rate of Application kg/ha | Pre- or Post-Emergence Application | Test Plants | | | | | |
|---|---|---|---|---|---|---|---|---|
| | | | Lt | To | Ot/Av | Ll | Cn | St |
| 25 | 10 | Pre | 2 | 2 | 3 | 1 | 0 | 3 |
| | | Post | 3 | 3 | 2 | 1 | 0 | 3 |

The names of the test plants are as follows:—

| | |
|---|---|
| Lt | Lettuce |
| To | Tomato |
| Ot/Av | Cultivated oats and wild oats (*Avena fatua*).<br>Wild oats are used in the post-emergence test and cultivated oats in the pre-emergence test. |
| Ll | *Lolium perenne* (perennial rye grass) |
| Cn | *Cyperus rotundus* |
| St | *Setaria viridis* |

EXAMPLE 4

This Example illustrates the herbicidal properties of the compounds of Table I. Tests were carried out as described in Example 3. The compound was formulated by mixing an appropriate amount of the compound with 5 ml of an emulsion prepared by diluting 160 ml of a solution containing 21.8 grams per litre of Span 80 and 78.2 grams per litre of Tween 20 in methylcyclohexanone to 500 ml with water. The mixture of the compound and emulsion was shaken with glass beads and diluted to 40 ml with water. Damage to plants was assessed on a scale of 0 to 5 where 0 is 0 to 20% damage and 5 is complete kill. In the table of results, a dash (—) means that no test was made. The results are given in Table III below:—

TABLE III

| Compound No. | A | B | Sb | Rp | Ct | Sy | Mz | Ww | Rc | Sn | Ip | Am | Pi | Ca | Po | Xs | Ab | Cv | Ot | Dg | Pu | St | Ec | Sh | Ag | Cn |
|---|---|---|---|---|---|---|---|---|---|---|---|---|---|---|---|---|---|---|---|---|---|---|---|---|---|---|
| 1 | Pre | 5.0 | 5 | 5 | 1 | 3 | 0 | 4 | 4 | 2 | 4 | 4 | 4 | 4 | 4 | 0 | 2 | – | 4 | 4 | 3 | 3 | 3 | 4 | 0 | 0 |
| | Post | 5.0 | 1 | 4 | 0 | 1 | 0 | 0 | 0 | 3 | 0 | 0 | 3 | 4 | 4 | 0 | 1 | 2 | 0 | 0 | 2 | 1 | 0 | 0 | 0 | 0 |
| 2 | Pre | 5.0 | 4 | 0 | 1 | 2 | 0 | 2 | 1 | 4 | 4 | 3 | 4 | 1 | 3 | 0 | 0 | – | 4 | 1 | 0 | 0 | 0 | 3 | 1 | 0 |
| | Post | 5.0 | 5 | 5 | 2 | 4 | 0 | 2 | 1 | 5 | 5 | 5 | 5 | 5 | 5 | 5 | 5 | 5 | 3 | 5 | 3 | 4 | 5 | 4 | 1 | 0 |
| 3 | Pre | 1.0 | 5 | 5 | 0 | 1 | 0 | 4 | 2 | – | 0 | 0 | 5 | 4 | 5 | 0 | 5 | – | 4 | 3 | 3 | 3 | 5 | 4 | 0 | 0 |
| | Post | 1.0 | 5 | 2 | 0 | 4 | 0 | 1 | 1 | 0 | 0 | 5 | 5 | 5 | 5 | 2 | 5 | 5 | 1 | 4 | 4 | 4 | 3 | 0 | 0 | 0 |
| 4 | Pre | 1.0 | 2 | 0 | 0 | 0 | 0 | 0 | 0 | – | 0 | 0 | 0 | 0 | 0 | 0 | 0 | – | 0 | 0 | 0 | 0 | 0 | 0 | 0 | 0 |
| | Post | 1.0 | 5 | 4 | 4 | 4 | 0 | 0 | 1 | 5 | 4 | 5 | 5 | 5 | 5 | 5 | 4 | 4 | 0 | 0 | – | 0 | 5 | 1 | 0 | 0 |
| 5 | Pre | 5.0 | 5 | 5 | 2 | 4 | 0 | 4 | 0 | – | 3 | 4 | – | 4 | 5 | 0 | 4 | – | 4 | – | 4 | 4 | 0 | 1 | 1 | 0 |
| | Post | 5.0 | 3 | 2 | 2 | 4 | 0 | 1 | 1 | 3 | 4 | 4 | 3 | 4 | 4 | 2 | 4 | 1 | 0 | 2 | 2 | 2 | 0 | 0 | 0 | 0 |
| 8 | Pre | 1.0 | 5 | 5 | 0 | 0 | 0 | 4 | 3 | – | 4 | 3 | 4 | – | 4 | 0 | 4 | – | 4 | – | 0 | 0 | 0 | 0 | 2 | 0 |
| | Post | 1.0 | 5 | 4 | 4 | 4 | 0 | 4 | 1 | 5 | 4 | 5 | 4 | 5 | 5 | 5 | 5 | – | 2 | 3 | 3 | 4 | 3 | 1 | 0 | 0 |
| | Pre | 5.0 | 5 | 5 | 5 | 3 | 0 | 4 | 3 | – | 5 | 4 | 4 | – | 5 | 5 | 5 | – | 4 | – | 2 | 2 | 1 | 4 | 4 | 0 |
| | Post | 5.0 | 5 | 5 | 4 | 5 | 0 | 4 | 1 | 5 | 4 | 5 | 5 | 5 | 5 | 5 | 5 | – | 3 | 3 | 3 | 4 | 4 | 4 | 2 | 0 |

TABLE III (Continued)

| Compound No. | A | B | Test Plants | | | | | | | | | | | | | | | | | | | | | | | |
|---|---|---|---|---|---|---|---|---|---|---|---|---|---|---|---|---|---|---|---|---|---|---|---|---|---|---|
| | | | Sb | Rp | Ct | Sy | Mz | Ww | Rc | Sn | Ip | Am | Pi | Ca | Po | Xs | Ab | Cv | Ot | Dg | Pu | St | Ec | Sh | Ag | Cn |
| 10 | Pre | 5.0 | 4 | 0 | 0 | 3 | 0 | 0 | 0 | – | 3 | 2 | 3 | 5 | 5 | 0 | 2 | – | 0 | – | 0 | 0 | 0 | 0 | 0 | 0 |
| | Post | 5.0 | 1 | 4 | 0 | 3 | 0 | 0 | 0 | 1 | 1 | 4 | 3 | 4 | 4 | 1 | 1 | 3 | 0 | 0 | 0 | 0 | 2 | 0 | 0 | 0 |
| 11 | Pre | 5.0 | 5 | 4 | 0 | 0 | 0 | 1 | 3 | – | 0 | 1 | 5 | – | 5 | 0 | 4 | – | 4 | – | 1 | 2 | 2 | 0 | 2 | 1 |
| | Post | 5.0 | 4 | 4 | 2 | 4 | 0 | 0 | 1 | 5 | 4 | 5 | 5 | 4 | 5 | 5 | 5 | 5 | 1 | 1 | 4 | 2 | 4 | 3 | 0 | 0 |
| 12 | Pre | 5.0 | 4 | 4 | 2 | 1 | 0 | 0 | 0 | – | 4 | 4 | 5 | 1 | 5 | 0 | 5 | – | 2 | – | 4 | 3 | 2 | 0 | 0 | 0 |
| | Post | 5.0 | 2 | 3 | 1 | 2 | 0 | 0 | 0 | 3 | 1 | 4 | 2 | 4 | 4 | 0 | 4 | – | 0 | 0 | 3 | 3 | 0 | 0 | 0 | 0 |
| 13 | Post | 1.0 | 5 | 4 | 3 | 5 | 0 | 2 | 1 | 5 | 5 | 5 | 5 | 5 | 5 | 5 | 4 | – | 4 | 2 | 2 | 3 | 3 | 4 | 2 | 2 |
| 14 | Pre | 1.0 | 5 | 5 | 4 | 0 | 0 | 1 | 1 | – | 0 | 0 | 5 | 0 | 5 | 0 | 4 | – | 4 | – | 0 | 0 | 0 | 0 | 1 | 1 |
| | Post | 1.0 | 4 | 5 | 2 | 3 | 0 | 1 | 0 | 5 | 5 | 5 | 4 | 5 | 5 | 5 | 5 | – | 2 | 3 | 2 | 4 | 4 | 1 | 0 | 0 |
| | Pre | 5.0 | 5 | 5 | 4 | 2 | 0 | 4 | 2 | – | 4 | 4 | 5 | 3 | 4 | 4 | 5 | – | 4 | – | 3 | 3 | 5 | 1 | 2 | 0 |
| | Post | 5.0 | 5 | 5 | 4 | 5 | 0 | 4 | 1 | 5 | 5 | 5 | 5 | 5 | 5 | 5 | 5 | – | 4 | 4 | 4 | 5 | 5 | 3 | 4 | 1 |
| 15 | Pre | 1.0 | 5 | 5 | 1 | 0 | 1 | 4 | 1 | – | 2 | 4 | 5 | 4 | 5 | 0 | 5 | – | 5 | – | 4 | 0 | 4 | 0 | 0 | 0 |
| | Post | 1.0 | 5 | 5 | 1 | 5 | 0 | 2 | 1 | 5 | 5 | 5 | 5 | 5 | 5 | 5 | 5 | – | 2 | 3 | 4 | 4 | 5 | 2 | 1 | 0 |

TABLE III (Continued)

| Compound No. | A | B | Sb | Rp | Ct | Sy | Mz | Ww | Rc | Sn | Ip | Am | Pi | Ca | Po | Xs | Ab | Cv | Ot | Dg | Pu | St | Ec | Sh | Ag | Cn |
|---|---|---|---|---|---|---|---|---|---|---|---|---|---|---|---|---|---|---|---|---|---|---|---|---|---|---|
| | | | Test Plants | | | | | | | | | | | | | | | | | | | | | | | |
| 16 | Pre | 5.0 | 4 | 4 | 1 | 1 | 1 | 0 | 0 | — | 0 | 4 | 3 | 0 | 4 | 0 | 1 | — | 3 | — | 2 | 0 | 0 | 0 | 0 | 0 |
| | Post | 5.0 | 0 | 4 | 1 | 1 | 0 | 1 | 0 | 1 | 4 | 5 | 2 | 5 | 5 | 0 | 1 | — | 0 | 2 | 3 | 2 | 0 | 0 | 0 | 0 |
| 17 | Pre | 5.0 | 5 | 5 | 4 | 4 | 1 | 4 | 2 | 5 | 4 | 5 | — | 4 | 5 | 3 | 5 | — | 5 | 4 | 4 | 5 | 5 | 3 | 3 | 0 |
| | Post | 5.0 | 5 | 3 | 4 | 4 | 1 | 1 | 1 | 5 | 3 | — | 3 | 4 | 5 | 3 | 3 | 4 | 1 | 2 | 5 | 4 | 1 | 2 | 1 | 0 |
| 19 | Pre | 5.0 | 5 | 5 | 4 | 4 | 3 | 4 | 2 | 5 | 5 | 4 | — | 4 | 5 | 4 | 5 | — | 4 | 5 | 4 | 5 | 5 | 4 | 1 | 0 |
| | Post | 5.0 | 5 | 5 | 1 | 4 | 2 | 2 | 2 | 4 | 4 | 5 | 5 | 4 | 5 | — | 5 | 5 | 3 | 4 | 4 | 4 | — | 0 | 2 | 0 |
| 20 | Pre | 5.0 | 5 | 5 | 4 | 4 | 4 | 4 | 3 | 5 | 5 | 4 | — | 5 | — | 0 | 5 | — | 4 | 4 | 4 | 5 | 5 | 5 | 4 | 0 |
| | Post | 5.0 | 5 | 5 | 5 | 5 | 5 | 5 | 5 | 5 | 5 | 5 | 5 | 5 | 5 | 5 | 5 | 5 | 5 | 5 | 5 | 5 | 5 | 5 | 4 | 3 |
| | Pre | 1.0 | 2 | 4 | 0 | 4 | 1 | 4 | 1 | 5 | 1 | 4 | — | 4 | — | 0 | 5 | — | 4 | 4 | 4 | 4 | 4 | 4 | 1 | 0 |
| | Post | 1.0 | 5 | 5 | 4 | 5 | 4 | 4 | 4 | 5 | 5 | 5 | 5 | 5 | 5 | 5 | 5 | 5 | 5 | 4 | 4 | 5 | 5 | 5 | 3 | 1 |
| 21 | Pre | 5.0 | 3 | 4 | 1 | 1 | 0 | 0 | 0 | 4 | 0 | 3 | 2 | 3 | — | 0 | 3 | — | 0 | 2 | 0 | 0 | 0 | — | 0 | 0 |
| | Post | 5.0 | 3 | 4 | 4 | 3 | 0 | 0 | 0 | 4 | 3 | — | 3 | 4 | — | 2 | 4 | 4 | 0 | — | 1 | 2 | 1 | — | 0 | 0 |
| 22 | Pre | 5.0 | 3 | 4 | 0 | 2 | 0 | 2 | 0 | 4 | 2 | 4 | — | 3 | — | — | 0 | — | 2 | 4 | 4 | 4 | 2 | 2 | 2 | 0 |
| | Post | 5.0 | 5 | 5 | 1 | 5 | 0 | 2 | — | 3 | 2 | 4 | 3 | 4 | — | — | 4 | 5 | 3 | 3 | 4 | 1 | 4 | 4 | 2 | 0 |

TABLE III (Continued)

| Compound No. | A | B | Test Plants | | | | | | | | | | | | | | | | | | | | | | |
|---|---|---|---|---|---|---|---|---|---|---|---|---|---|---|---|---|---|---|---|---|---|---|---|---|---|
| | | | Sb | Rp | Ct | Sy | Mz | Ww | Rc | Sn | Ip | Am | Pi | Ca | Po | Xs | Ab | Cv | Ot | Dg | Pu | St | Ec | Sh | Ag | Cn |
| 23 | Pre | 5.0 | 4 | 4 | 0 | 1 | 0 | 2 | 0 | 4 | 2 | 4 | – | 4 | – | – | 4 | – | 2 | 4 | 3 | 3 | 2 | 1 | 0 | 0 |
| | Post | 5.0 | 4 | 4 | 2 | 4 | 1 | 3 | – | 3 | 3 | 3 | 2 | 4 | – | – | 4 | 2 | 3 | 1 | 3 | 2 | 4 | 5 | 1 | 0 |
| 24 | Pre | 5.0 | 4 | 5 | 0 | 1 | 0 | 2 | 0 | 4 | 3 | 2 | – | 2 | – | – | 4 | – | 3 | 4 | 2 | 2 | 4 | 1 | 1 | 0 |
| | Post | 5.0 | 5 | 5 | 4 | 5 | 1 | 5 | – | 5 | 5 | 5 | 5 | 5 | – | – | 5 | 5 | 5 | 5 | 5 | 5 | 5 | 5 | 4 | 1 |
| 25 | Pre | 5.0 | 2 | 3 | 0 | 0 | 0 | 0 | 0 | 4 | 3 | 0 | – | 3 | – | 0 | 1 | – | 2 | 2 | 0 | 2 | 0 | 0 | 0 | 0 |
| | Post | 5.0 | 3 | 3 | 0 | 2 | 0 | 1 | 0 | 3 | 4 | 2 | 1 | 4 | – | – | 1 | 4 | 0 | 0 | 2 | 1 | 2 | 2 | 0 | 0 |
| 27 | Pre | 5.0 | 5 | 5 | 2 | 3 | 0 | 1 | 0 | 1 | 1 | 2 | – | 4 | 4 | 0 | 4 | – | 4 | – | 4 | 3 | 4 | 0 | 2 | 0 |
| | Post | 5.0 | 2 | 0 | 0 | 0 | 1 | 0 | 0 | 1 | 0 | 2 | 3 | 0 | 1 | 0 | 0 | 0 | 0 | 0 | 2 | 0 | 0 | 0 | 0 | 0 |
| 29 | Pre | 5.0 | 4 | 4 | 1 | 2 | 0 | 1 | 0 | 4 | 2 | 4 | – | 3 | 5 | 2 | 2 | – | 1 | 4 | 4 | 3 | 3 | 0 | 0 | 1 |
| | Post | 5.0 | 1 | 1 | 1 | 2 | 0 | 0 | 0 | 3 | 1 | – | 3 | 1 | 4 | 0 | 2 | 3 | 0 | 1 | 1 | 0 | 0 | 0 | 0 | 0 |
| 31 | Pre | 3.5 | 4 | 5 | 0 | 3 | 0 | 2 | 0 | 3 | 2 | 3 | – | 4 | 5 | 0 | 5 | – | 3 | 4 | 4 | 4 | 4 | 2 | 1 | 0 |
| | Post | 3.5 | 5 | 5 | 4 | 5 | 0 | 2 | 1 | 5 | 5 | – | 5 | 5 | 5 | 5 | 5 | 5 | 1 | 4 | 5 | 5 | 5 | 3 | 2 | 2 |
| 32 | Pre | 5.0 | 5 | 5 | 0 | 2 | 0 | 1 | 0 | 3 | 1 | 3 | – | 4 | 5 | 0 | 4 | – | 3 | 5 | 4 | 3 | 4 | 3 | 2 | 0 |
| | Post | 5.0 | 5 | 5 | 4 | 5 | 0 | 1 | 1 | 5 | 4 | – | 5 | 5 | 5 | 5 | 5 | 5 | 1 | 5 | 5 | 4 | 3 | 3 | 3 | 2 |

TABLE III (Continued)

| Compound No. | A | B | Test Plants | | | | | | | | | | | | | | | | | | | | | | |
|---|---|---|---|---|---|---|---|---|---|---|---|---|---|---|---|---|---|---|---|---|---|---|---|---|---|
| | | | Sb | Rp | Ct | Sy | Mz | Ww | Rc | Sn | Ip | Am | Pi | Ca | Po | Xs | Ab | Cv | Ot | Dg | Pu | St | Ec | Sh | Ag | Cn |
| 33 | Pre | 5.0 | 5 | 2 | 0 | 1 | 0 | 0 | 0 | 4 | 2 | 2 | – | 3 | 5 | 2 | 2 | – | 1 | 1 | 0 | 0 | 0 | 0 | 0 | 0 |
| | Post | 5.0 | 5 | 5 | 2 | 4 | 0 | 0 | 0 | 5 | 5 | – | 5 | 4 | 5 | – | 4 | 5 | 0 | 0 | 2 | 0 | 4 | 0 | 0 | 0 |
| 34 | Pre | 5.0 | 4 | 4 | 1 | 1 | 0 | 0 | 0 | 4 | 4 | 2 | 4 | 3 | 4 | – | 4 | – | 0 | 3 | 3 | 3 | 2 | 0 | 0 | 0 |
| | Post | 5.0 | 3 | 1 | 1 | 0 | 0 | 0 | 0 | 2 | 2 | 4 | 1 | 4 | 5 | 0 | 2 | 0 | 0 | 0 | 1 | 0 | 0 | 2 | 0 | 0 |
| 35 | Pre | 5.0 | 5 | 4 | 3 | 4 | 1 | 0 | 0 | 2 | 0 | 4 | – | 0 | 4 | 2 | 5 | – | 1 | 4 | 3 | 3 | 3 | 2 | 0 | 1 |
| | Post | 5.0 | 2 | 3 | 0 | 3 | 0 | 0 | 0 | 4 | 0 | – | 3 | 0 | 4 | 2 | 2 | 5 | 0 | 0 | 2 | 2 | 2 | 0 | 0 | 2 |
| 36 | Pre | 5.0 | 5 | 4 | 3 | 4 | 1 | 0 | 0 | 2 | 0 | 4 | – | 0 | 4 | 2 | 5 | – | 1 | 4 | 3 | 3 | 3 | 2 | 0 | 1 |
| | Post | 5.0 | 2 | 3 | 0 | 3 | 0 | 0 | 0 | 4 | 0 | – | 3 | 0 | 4 | 2 | 2 | 5 | 0 | 0 | 2 | 2 | 2 | 0 | 0 | 2 |
| 38 | Pre | 5.0 | 5 | 5 | 1 | 0 | 1 | 3 | 1 | 4 | 3 | 1 | – | 4 | 4 | 4 | 4 | – | 4 | 5 | 4 | 2 | 4 | 3 | 2 | 0 |
| | Post | 5.0 | 5 | 1 | 2 | 1 | 1 | 1 | 0 | 1 | 0 | 5 | 5 | 5 | 5 | 5 | 4 | 3 | 1 | 5 | 5 | 5 | 4 | 3 | 1 | 0 |
| 39 | Pre | 3.0 | 2 | 2 | 1 | 0 | 0 | 0 | 0 | 4 | 2 | 3 | – | 2 | 3 | 4 | 3 | – | 0 | 2 | 3 | 2 | 1 | 0 | 2 | 0 |
| | Post | 3.0 | 3 | 5 | 0 | 5 | 0 | 0 | 1 | 5 | 2 | – | 4 | 4 | 5 | – | 5 | 5 | 1 | 4 | 4 | 1 | 2 | 2 | 0 | 0 |
| 43 | Pre | 5.0 | 3 | 3 | 3 | 3 | 0 | 4 | 1 | 4 | 0 | 5 | 5 | 4 | 4 | – | 4 | – | 4 | 4 | 4 | 5 | 2 | 0 | 0 | 0 |
| | Post | 5.0 | 5 | 5 | 4 | 4 | 0 | 3 | 2 | 5 | 4 | 1 | 5 | 5 | 2 | 0 | 4 | 5 | 5 | 1 | 4 | 3 | 3 | 0 | 2 | 1 |

TABLE III (Continued)

| Compound No. | A | B | Test Plants | | | | | | | | | | | | | | | | | | | | | | | |
|---|---|---|---|---|---|---|---|---|---|---|---|---|---|---|---|---|---|---|---|---|---|---|---|---|---|---|
| | | | Sb | Rp | Ct | Sy | Mz | Ww | Rc | Sn | Ip | Am | Pi | Ca | Po | Xs | Ab | Cv | Ot | Dg | Pu | St | Ec | Sh | Ag | Cn |
| 44 | Pre | 5.0 | 5 | 5 | 2 | 2 | 0 | 4 | 2 | 4 | 0 | 3 | 5 | 4 | 5 | — | 3 | — | 4 | 4 | 4 | 4 | 4 | 0 | 0 | 0 |
| | Post | 5.0 | 5 | 5 | 3 | 3 | 0 | 4 | 4 | 2 | 3 | 0 | 4 | 4 | 4 | 5 | 4 | 5 | 4 | 5 | 4 | 5 | 5 | 5 | 3 | 0 |
| 45 | Post | 5.0 | 4 | 5 | 0 | 3 | 0 | 1 | 0 | 4 | 2 | 0 | 4 | 4 | 4 | 4 | 4 | 5 | 1 | 0 | 1 | 1 | 2 | 0 | 0 | 0 |
| 46 | Pre | 1.0 | 0 | 1 | 0 | 0 | 0 | 0 | 0 | 3 | 0 | 0 | 0 | 3 | 2 | 3 | 1 | — | 0 | 0 | 2 | 0 | 0 | 0 | 0 | 0 |
| | Post | 1.0 | 3 | 0 | 0 | 3 | 0 | 0 | 0 | 2 | 0 | 2 | 1 | 2 | 0 | 0 | 1 | 0 | 0 | 0 | 0 | 0 | 0 | 0 | 0 | 1 |
| 47 | Pre | 5.0 | 3 | 4 | 0 | 0 | 0 | 0 | 0 | 4 | 0 | 0 | 2 | 0 | 0 | — | 4 | — | 2 | 0 | 0 | 1 | 0 | 0 | 1 | 0 |
| | Post | 5.0 | 2 | 5 | 0 | 5 | 0 | 1 | 0 | 5 | 4 | 0 | 3 | 5 | 5 | 5 | 4 | 5 | 0 | 3 | 0 | 1 | 3 | 0 | 0 | 0 |
| 48 | Pre | 1.0 | 4 | 5 | 2 | 1 | 0 | 3 | 2 | 5 | 1 | 4 | 3 | 4 | 5 | 0 | 5 | — | 4 | 4 | 4 | 3 | 3 | 0 | 0 | 0 |
| | Post | 1.0 | 5 | 3 | 0 | 4 | 1 | 2 | 1 | 3 | 3 | 3 | 3 | 4 | 4 | 3 | 3 | 4 | 3 | 3 | 4 | 3 | 3 | 4 | 3 | 0 |
| 49 | Pre | 3.0 | 5 | 5 | 2 | 4 | 0 | 5 | 4 | 4 | 4 | 0 | 5 | 4 | 5 | 3 | 4 | — | 5 | 4 | 4 | 4 | 5 | 2 | 2 | 0 |
| | Post | 3.0 | 5 | 5 | 4 | 5 | 0 | 4 | 5 | 5 | 5 | 4 | 5 | 5 | 5 | 5 | 5 | 5 | 5 | 5 | 4 | 5 | 5 | 5 | 4 | 3 |
| 50 | Pre | 5.0 | 4 | 4 | 0 | 1 | 0 | 4 | 2 | 5 | 0 | 0 | 5 | 3 | 5 | — | 0 | — | 4 | 4 | 4 | 4 | 5 | 0 | 2 | 2 |
| | Post | 5.0 | 5 | 5 | 0 | 5 | 1 | 3 | 3 | 4 | 3 | 2 | 5 | 5 | 5 | 5 | 4 | 5 | 3 | 4 | 4 | 4 | 4 | 5 | 2 | 0 |

TABLE III (Continued)

| Compound No. | A | B | Test Plants | | | | | | | | | | | | | | | | | | | | | | |
|---|---|---|---|---|---|---|---|---|---|---|---|---|---|---|---|---|---|---|---|---|---|---|---|---|---|
| | | | Sb | Rp | Ct | Sy | Mz | Ww | Rc | Sn | Ip | Am | Pi | Ca | Po | Xs | Ab | Cy | Ot | Dg | Pu | St | Ec | Sh | Ag | Cn |
| 51 | Pre | 5.0 | 4 | 4 | 0 | 3 | 0 | 1 | 1 | 5 | — | — | 4 | 4 | 5 | 0 | 5 | — | 1 | 4 | 2 | 4 | 1 | 0 | 0 | 0 |
| | Post | 5.0 | 3 | 3 | 0 | 3 | 2 | 1 | 0 | 1 | 3 | 2 | 2 | 4 | 4 | 0 | 3 | 5 | 0 | 3 | 1 | 1 | 1 | 0 | 0 | 0 |
| 52 | Pre | 5.0 | 5 | 5 | 5 | 4 | 5 | 5 | 4 | 4 | 4 | 5 | 5 | 4 | 5 | — | 5 | — | 5 | 5 | 5 | 5 | 5 | 4 | 3 | 0 |
| | Post | 5.0 | 5 | 5 | 4 | 5 | 5 | 4 | 5 | 5 | 5 | 5 | 5 | 5 | 5 | 5 | 5 | 5 | 5 | 5 | 5 | 5 | 5 | 5 | 5 | 2 |
| 53 | Pre | 5.0 | 5 | 5 | 1 | 0 | 0 | 4 | 3 | 2 | 5 | 0 | 3 | 4 | 0 | 3 | 5 | — | 5 | 4 | 5 | 4 | 5 | 4 | 2 | 0 |
| | Post | 5.0 | 5 | 5 | 4 | 4 | 0 | 5 | 5 | 5 | 5 | 2 | 5 | 5 | 5 | 5 | 5 | 5 | 5 | 4 | 5 | 5 | 5 | 5 | 4 | 0 |
| 54 | Pre | 5.0 | 4 | 4 | 3 | 3 | 1 | 3 | 0 | 4 | 3 | 2 | 5 | 4 | — | — | 4 | — | 3 | 4 | 4 | 4 | 3 | 2 | 0 | 0 |
| | Post | 5.0 | 5 | 5 | 4 | 5 | 1 | 4 | 3 | 5 | 5 | 5 | 5 | 5 | 5 | 5 | 4 | 4 | 5 | — | 4 | 3 | 4 | 5 | 1 | 0 |
| 55 | Pre | 3.0 | 2 | 3 | 0 | 0 | 0 | 3 | 0 | 1 | 0 | 2 | 2 | 0 | 0 | — | 1 | — | 3 | 4 | 4 | 3 | 1 | 0 | 0 | ● |
| | Post | 3.0 | 3 | 2 | 0 | 0 | 1 | 2 | 0 | 1 | 5 | 1 | 3 | 4 | 5 | 3 | 1 | 3 | 2 | — | 3 | 1 | 2 | 4 | 2 | 0 |
| 56 | Pre | 5.0 | 5 | 5 | 4 | 4 | 2 | 5 | 4 | 1 | 0 | 5 | 5 | 4 | 5 | 4 | 5 | — | 5 | 4 | 5 | 5 | 5 | 3 | 4 | 0 |
| | Post | 5.0 | 5 | 5 | 4 | 5 | 5 | 4 | 5 | 5 | 1 | 5 | 5 | 5 | 5 | 5 | 5 | 5 | 5 | 5 | 5 | 5 | 5 | 5 | 5 | 3 |
| 57 | Pre | 5.0 | 5 | 3 | 1 | 1 | 1 | 3 | 2 | 3 | 2 | 4 | 5 | 4 | 4 | 0 | 4 | — | 3 | 3 | 4 | 2 | 2 | 1 | 0 | 0 |
| | Post | 5.0 | 5 | 5 | 4 | 5 | 1 | 4 | 4 | 5 | 4 | 4 | 5 | 5 | 5 | 5 | 5 | 5 | 5 | 4 | 5 | 5 | 5 | 3 | 4 | 1 |

TABLE III (Continued)

| Compound No. | A | B | Test Plants | | | | | | | | | | | | | | | | | | | | | | | |
|---|---|---|---|---|---|---|---|---|---|---|---|---|---|---|---|---|---|---|---|---|---|---|---|---|---|---|
| | | | Sb | Rp | Ct | Sy | Mz | Ww | Rc | Sn | Ip | Am | Pi | Ca | Po | Xs | Ab | Cv | Ot | Dg | Pu | St | Ec | Sh | Ag | Cn |
| 58 | Pre | 5.0 | 5 | 4 | 4 | 4 | 2 | 4 | 1 | 4 | 4 | 5 | 5 | 4 | 5 | – | 5 | – | 4 | 4 | 4 | 5 | 2 | 1 | 0 | 0 |
| | Post | 5.0 | 4 | 5 | 3 | 4 | 2 | 4 | 3 | 5 | 4 | 3 | 5 | 5 | 5 | 5 | 5 | 5 | 5 | 3 | 5 | 4 | 4 | 3 | 4 | 1 |
| 59 | Pre | 5.0 | 4 | 2 | 2 | 3 | 0 | 4 | 3 | 1 | 0 | 0 | 3 | 4 | 2 | 0 | 4 | – | 4 | 4 | 4 | 5 | 3 | 2 | 2 | 0 |
| | Post | 5.0 | 5 | 4 | 3 | 5 | 0 | 4 | 5 | 4 | 3 | 3 | 5 | 5 | 5 | 4 | 5 | 5 | 5 | 5 | 5 | 5 | 5 | 3 | 3 | 1 |
| 60 | Pre | 5.0 | 5 | 4 | 2 | 0 | 0 | 1 | 0 | 3 | 4 | 0 | 2 | 4 | 3 | 0 | 2 | – | 2 | 0 | 3 | 2 | 1 | 0 | 1 | 0 |
| | Post | 5.0 | 3 | 3 | 1 | 2 | 0 | 2 | 0 | 3 | 4 | 0 | 2 | 4 | 3 | 0 | 4 | 4 | 1 | 2 | 2 | 1 | 0 | 0 | 2 | 0 |
| 61 | Pre | 5.0 | 5 | 5 | 4 | 3 | 0 | 5 | 4 | 0 | 1 | 2 | 5 | 4 | 3 | – | 5 | – | 5 | 4 | 5 | 5 | 5 | 2 | 2 | 0 |
| | Post | 5.0 | 5 | 5 | 3 | 4 | 2 | 5 | 5 | 5 | 4 | 4 | 5 | 5 | 3 | 5 | 5 | 5 | 4 | 5 | 5 | 5 | 5 | 4 | 4 | 3 |
| 62 | Post | 5.0 | 5 | 5 | 0 | 2 | 0 | 2 | 0 | 5 | 5 | 2 | 5 | 5 | 4 | 5 | 4 | 4 | 3 | 0 | 0 | 0 | 2 | 0 | 1 | 0 |
| 63 | Pre | 4.5 | 3 | 3 | 1 | 1 | 0 | 1 | 0 | 4 | 1 | 4 | 3 | 4 | 5 | – | 4 | – | 2 | 3 | 4 | 4 | 0 | 0 | 0 | 0 |
| | Post | 4.5 | 2 | 1 | 1 | 1 | 0 | 0 | 0 | 3 | 1 | 0 | 2 | 4 | 1 | 0 | 3 | 2 | 0 | 0 | 1 | 0 | 1 | 0 | 0 | 0 |
| 65 | Pre | 5.0 | 4 | 1 | 0 | 0 | 0 | 0 | 0 | 3 | 0 | 2 | 3 | 4 | 5 | 0 | 0 | – | 2 | 0 | 0 | 0 | 0 | 0 | 0 | 0 |
| | Post | 5.0 | 5 | 3 | 0 | 3 | 0 | 4 | 5 | 5 | 3 | 4 | 3 | 4 | 2 | 3 | 5 | 5 | 4 | 2 | 0 | 4 | 0 | 0 | 0 | 0 |

TABLE III (Continued)

| Compound No. | A | B | Test Plants | | | | | | | | | | | | | | | | | | | | | | |
|---|---|---|---|---|---|---|---|---|---|---|---|---|---|---|---|---|---|---|---|---|---|---|---|---|---|
| | | | Sb | Rp | Ct | Sy | Mz | Ww | Rc | Sn | Ip | Am | Pi | Ca | Po | Xs | Ab | Cv | Ot | Dg | Pu | St | Ec | Sh | Ag | Cn |
| 66 | Pre | 5.0 | 5 | 5 | 5 | 4 | 2 | 4 | 3 | 5 | 4 | 5 | 5 | 4 | 5 | — | 5 | — | 5 | 5 | 5 | 5 | 5 | 1 | 1 | 0 |
| | Post | 5.0 | 5 | 5 | 4 | 5 | 3 | 5 | 5 | 5 | 5 | 5 | 5 | 5 | 5 | 5 | 5 | 5 | 5 | 4 | 5 | 5 | 5 | 3 | 4 | 2 |
| 67 | Pre | 5.0 | 5 | 4 | 1 | 0 | 2 | 3 | 2 | 2 | 4 | 0 | 3 | 3 | 3 | 0 | 5 | — | 4 | 4 | 3 | 2 | 3 | 0 | 0 | 0 |
| | Post | 5.0 | 5 | 3 | 0 | 3 | 0 | 3 | 2 | 1 | 4 | 0 | 3 | 5 | 0 | 0 | 3 | 4 | 3 | 1 | 3 | 2 | 2 | 0 | 2 | 0 |
| 68 | Pre | 1.0 | 3 | 4 | 2 | 3 | 0 | 4 | 0 | 2 | 3 | 4 | 5 | 4 | 4 | 0 | 4 | — | 4 | 4 | 4 | 4 | 2 | 0 | 1 | 0 |
| | Post | 1.0 | 2 | 5 | 0 | 3 | 0 | 4 | 3 | 5 | 5 | 5 | 5 | 5 | 5 | 1 | 4 | 5 | 4 | 1 | 4 | 4 | 3 | 3 | 3 | 0 |
| | Pre | 5.0 | 5 | 5 | 4 | 4 | 2 | 4 | 3 | 3 | 5 | 4 | 5 | 4 | 4 | 0 | 5 | — | 5 | 5 | 5 | 5 | 5 | 3 | 3 | 0 |
| | Post | 5.0 | 5 | 5 | 4 | 5 | 2 | 4 | 5 | 5 | 5 | 5 | 5 | 5 | 5 | 5 | 5 | 5 | 5 | 5 | 5 | 5 | 5 | 5 | 4 | 3 |
| 69 | Pre | 5.0 | 5 | 4 | 0 | 4 | 2 | 4 | 3 | 2 | 0 | 4 | 5 | 4 | 5 | — | 5 | — | 4 | 5 | 5 | 4 | 3 | 0 | 1 | 0 |
| | Post | 5.0 | 5 | 5 | 0 | 4 | 0 | 4 | 3 | 5 | 0 | 5 | 5 | 5 | 3 | 2 | 5 | 5 | 4 | 2 | 4 | 5 | 4 | 0 | 3 | 0 |
| 70 | Pre | 5.0 | 2 | 3 | 1 | 0 | 0 | 2 | 0 | 4 | 1 | 2 | 5 | 4 | 4 | — | 5 | — | 1 | 4 | 4 | 3 | 2 | 0 | 0 | 0 |
| | Post | 5.0 | 3 | 3 | 1 | 4 | 0 | 3 | 1 | 4 | 3 | 0 | 4 | 5 | 4 | 1 | 4 | 3 | 1 | 0 | 2 | 3 | 1 | 1 | 2 | 0 |
| 72 | Pre | 5.0 | 4 | 5 | 0 | 0 | 0 | 0 | 0 | 0 | 0 | 0 | 3 | 4 | 5 | 0 | 1 | — | 3 | 0 | 3 | 1 | 1 | 0 | 0 | 0 |
| | Post | 5.0 | 0 | 0 | 0 | 0 | 0 | 0 | 0 | 1 | 0 | 2 | 5 | 2 | 4 | 0 | 0 | 3 | 0 | 0 | 1 | 1 | 0 | 0 | 0 | 0 |

TABLE III. (Continued)

| Compound No. | A | B | Test Plants | | | | | | | | | | | | | | | | | | | | | | | |
|---|---|---|---|---|---|---|---|---|---|---|---|---|---|---|---|---|---|---|---|---|---|---|---|---|---|---|
| | | | Sb | Rp | Ct | Sy | Mz | Ww | Rc | Sn | Ip | Am | Pi | Ca | Po | Xs | Ab | Cv | Ot | Dg | Pu | St | Ec | Sh | Ag | Cn |
| 73 | Pre | 5.0 | 5 | 0 | 2 | 2 | 0 | 0 | 0 | 3 | 0 | 2 | — | 1 | 2 | 0 | 2 | — | 0 | 0 | 2 | 0 | 0 | 0 | 0 | 0 |
| | Post | 5.0 | 3 | 4 | 1 | 3 | 0 | 0 | 0 | 5 | 2 | — | 4 | 3 | 4 | 5 | 4 | 5 | 0 | 0 | 4 | 3 | 1 | 0 | 0 | 0 |

The names of the test plants were as follows:—

| | |
|---|---|
| Sb | Sugar beet |
| Rp | Rape |
| Ct | Cotton |
| Sy | Soya bean |
| Mz | Maize |
| Ww | Winter wheat |
| Rc | Rice |
| Sn | *Senecio vulgaris* |
| Ip | *Ipomoea purpurea* |
| Am | *Amaranthus retroflexus* |
| Pi | *Polygonum aviculare* |
| Ca | *Chenopodium album* |
| Po | *Portulaca oleracea* |
| Xa | *Xanthium pensylvanicum* |
| Ab | *Abutilon theophrastii* |
| Cv | *Convolvulus arvensis* |
| Ot | Cultivated oats and wild oats (*Avena fatua*) Wild oats are used in the post-emergence test and cultivated oats in the pre-emergence test. |
| Dg | *Digitaria sanguinalis* |
| Pu | *Poa annua* |
| St | *Setaria viridis* |
| Ec | *Echinochloa crus-galli* |
| Sh | *Sorghum halepense* |
| Ag | *Agropyron repens* |
| Cn | *Cyperus rotundus* |

EXAMPLE 5

This Example illustrates the preparation of compound 14 of Table I. Compound 1 of Table I (3.0 g) was suspended in carbon tetrachloride (125 ml). Sulphuryl chloride (2.5 g) was added dropwise. When addition was complete, the mixture was heated under reflux for 4 hours, and then cooled. The solid was collected and recrystallised from toluene to give compound no 14 (1.6 g) with a melting point of 162°C.

EXAMPLE 6

This Example illustrates the preparation of compound no 13 of table I. Compound 1 of Table I (3.0 g) was added in portions to concentrated sulphuric acid (15 g), with stirring and cooling. Fuming nitric acid (5 g) was added dropwise with stirring and cooling to below 10°C. The mixture was then stirred for 30 minutes at room temperature and then heated for 1 hour on a steam both. The resulting solution was cooled and poured on to ice. The yellow solid was recrystallised from toluene to give compound no 13 (2.5 g) with a melting point of 172°C.

EXAMPLE 7

This Example illustrates the preparation of compound no 8 of Table I by a method alternative to that of Example 2.

3-Amino-4-bromopyrazole (1.62 g) was added in portions to a stirred solution of isopropyl bis-chloroformylamine (1.85 g) in dry acetonitrile. When addition was complete the mixture was heated under reflux for 2 hours. The mixture was cooled and filtered. Evaporation of the filtrate gave a solid (0.5 g) identified as compound no 8 by its melting point (214°C) and its nuclear magnetic resonance spectrum and mass spectrum.

EXAMPLE 8

This Example illustrates the preparation of compound no 2 of Table I.

3-Amino-4-cyanopyrazole (3.24 g) was added in portions with stirring at room temperature to a solution of isopropyl bis-chloroformylamine (5.6 g) in dry acetonitrile (60 ml). The mixture was then heated under reflux for 4 hours, cooled, and filtered. The filtrate was evaporated to give a gummy solid which was extracted with boiling toluene (125 ml). The extract was left overnight to cool and the crystals which separated (2.25 g) collected and identified as compound no 2 with a melting point of 132°C.

EXAMPLE 9

This Example illustrates the preparation of compound no 10 of Table I.

3-Amino-4-cyano-5-methoxypyrazole (2.0 g) was added in portions to a stirred solution of isopropyl bischloroformylamine in dry acetonitrile. After addition was complete the mixture was heated under reduced pressure and the residue triturated with ethyl acetate. The ethyl acetate solution was

filtered and evaporated to give a gummy solid which was triturated with water. The solid so obtained (0.8 g) was identified as compound no 10 of Table I.

EXAMPLE 10

This Example illustrates the preparation of compound no 9 of Table I.

3-Amino-4-cyano-5-dimethylaminopyrazole (1.85 g) was added in portions to a stirred solution of isopropyl bischloroformylamine (2.3 g) in dry acetonitrile (30 ml). The mixture was heated under reflux for 2½ hours, cooled, and filtered. The filtrate was evaporated. The gummy solid which remained was triturated with ether, and collected to give compound 9 (1.3 g) with a melting point of 200°C.

EXAMPLE 11

This Example illustrates the preparation of compound no 7 of Table I.

Compound no 6 of Table I (0.9 g), prepared from 3-amino-4-cyano-5-methylthiopyrazole and isopropyl bischloroformylamine in the usual way, was suspended in 90% sulphuric acid (10 ml) and heated to 90—100°C for 1 hour, cooled, and poured into ice and water. The white solid which resulted was collected and dried to give compound no 7 (0.8 g) with a melting point of 260°C.

EXAMPLE 12

This Example illustrates the preparation of compound no 60 of Table I.

Compounds no 57 of Table I (0.95 g) was added in portions with stirring to a suspension of sodium hydride (0.08 g) in dry dimethylformamide (20 ml). When effervescence had ceased, the clear solution was treated dropwise with methyl iodide (0.5 g) in a small volume of dry dimethylformamide. The mixture was then stirred for 1 hour and poured into ice and water (75 g). The resulting solid was collected, dried, and identified as compound 60 (0.83 g) with a melting point of 129°C.

**Claims**

1. Herbicidal pyrazolotriazinedione compounds of the formula (I)

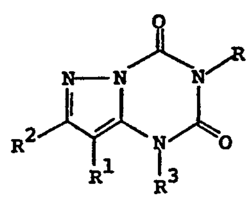

(I)

and tautomers and salts thereof, wherein R is a cycloalkyl or branched alkyl group of 3 to 10 carbon atoms, $R^1$ is hydrogen, lower alkyl, chlorine, fluorine, bromine, iodine, cyano, nitro, carbamoyl, or lower alkanoylamido; $R^2$ is hydrogen, or lower alkyl, lower mono- or di-alkylamino, lower alkoxy, or lower alkylthio; and $R^3$ is hydrogen or lower alkyl.

2. Compounds as claimed in claim 1 wherein $R^1$ is a halogen atom or a methyl or cyano group.

3. Compounds as claimed in claim 1 or claim 2 wherein R is an isopropyl, 1-methylpropyl, *iso*-butyl, *cyclo*pentyl, or cyclohexyl group.

4. The compound as claimed in claim 1 wherein R is isopropyl, $R^1$ is chlorine, $R^2$ is hydrogen and $R^3$ is hydrogen.

5. Herbicidal compositions comprising as an active ingredient a compound of the formula (I) as defined in claim 1, in admixture with a carrier comprising a solid or liquid diluent.

6. Herbicidal compositions comprising at least one compound of the formula (I) as defined in claim 1, in admixture with at least one other herbicide not of the formula (I).

7. A process of inhibiting the growth of unwanted plants which comprises applying to the plants or to the locus thereof, a compound of the formula (I) as defined in claim 1, in a herbicidally effective amount.

8. A process as claimed in claim 7 wherein the rate of application of the compound of formula (I) is from 0.5 to 10 kilograms per hectare.

9. A process of preparing compounds of the formula (I) defined in claim 1 wherein R, $R^1$ and $R^2$ are defined as in claim 1 and $R^3$ is hydrogen, which comprises reacting an alkyl or cycloalkyl bis-chloroformylamine of formula $RN(COCl)_2$ with a 3-amino-4,5-$R^1R^2$-disubstituted pyrazole and recovering the compound of formula (I) wherein $R^3$ is hydrogen.

10. A process of preparing compounds of the formula (I) wherein the group $R^1$ is bromine, chlorine, iodine, or nitro, which comprises reacting a compound of the formula (I) wherein the group $R^1$ is hydrogen with a halogenating or nitrating agent.

## Revendications

1. Pyrazolotriazinediones herbicides de formule:

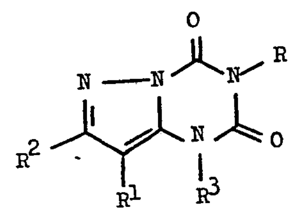

(I)

et leurs tautomères et sels, formule où R représente un radical cycloalkyle ou alkyle ramifié de 3 à 10 atomes de carbone, $R^1$ représente un atome d'hydrogène, de chlore, de fluor, de brome ou d'iode ou un radical alkyle inférieur, cyano, nitro, carbamoyle ou alkanoylamido inférieur, $R^2$ représente un un atome d'hydrogène ou un radical alkyle inférieur, mono(alkyl inférieur)amino, di(alkyl inférieur)amino, alkoxy inférieur ou alkylthio inférieur, et $R^3$ représente un atome d'hydrogène ou un radical alkyle inférieur.

2. Composés suivant la revendication 1, dans la formule desquels $R^1$ représente un atome d'halogène ou un radical méthyle ou cyano.

3. Composés suivant la revendication 1 ou 2, dans la formule desquels R représente un radical isopropyle, 1-méthylpropyle, isobutyle, cyclopentyle ou cyclohexyle.

4. Composé suivant la revendication 1, dans la formule duquel R représente un radical isopropyle, $R^1$ représente un atome de chlore, $R^2$ représente un atome d'hydrogène et $R^3$ représente un atome d'hydrogène.

5. Compositions herbicides, caractérisées en ce qu'elles comprennent comme constituant actif un composé de formule (I) suivant la revendication 1 en mélange avec un excipient comprenant un diluant solide ou liquide.

6. Compositions herbicides, caractérisées en ce qu'elles comprennent au moins un composé de formule (I) suivant la revendication 1 en mélange avec au moins un autre herbicide ne répondant à la formule (I).

7. Procédé pour inhiber la croissance d'espèces végétales indésirables, caractérisé en ce qu'on applique sur les plantes ou leur lieu de croissance un composé de formule (I) suivant la revendication 1 en quantité herbicide efficace.

8. Procédé suivant la revendication 7, caractérisé en ce que la dose d'application du composé de formule (I) est de 0,5 à 10 kg par hectare.

9. Procédé de préparation de composés de formule (I) suivant la revendication 1 où R, $R^1$ et $R^2$ ont les significations qui leur ont été données dans la revendication 1 et $R^3$ représente un atome d'hydrogène, caractérisé en ce qu'on fait réagir une alkyl-bis-chloroformylamine ou cycloalkyl-bis-chloroformylamine de formule $RN(COCl)_2$ avec un pyrazole 3-amino-4,5-$R^1R^2$-disubstitué et on recueille le composé de formule (I) où $R^3$ représente un atome d'hydrogène.

10. Procédé de préparation de composés de formule (I) où $R^1$ représente un atome de brome, de chlore, ou d'iode ou un radical nitro, caractérisé en ce qu'on fait réagir un composé de formule (I) où $R^1$ représente un atome d'hydrogène avec un agent d'halogénation ou un agent de nitration.

## Patentansprüche

1. Herbicide Pyrazolotriazindion-Verbindungen der Formel

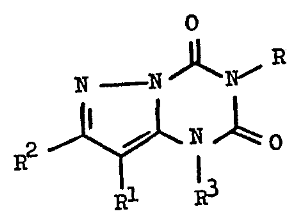

(I)

sowie Tautomere und Salze davon, worin R eine Cycloalkyl- oder verzweigte Alkylgruppe mit 3 bis 10 Kohlenstoffatomen ist; $R^1$ Wasserstoff, Niederalkyl, Chlor, Fluor, Brom, Jod, Cyano, Nitro, Car bamoyl oder Niederalkanoylamido ist; $R^2$ Wasserstoff oder Niederalkyl, Mono- oder Diniederalkylamino, Niederalkoxy oder Niederalkylthio ist; und $R^3$ Wasserstoff oder Niederalkyl ist.

2. Verbindungen nach Anspruch 1, worin $R^1$ ein Halogenatom oder eine Methyloder Cyanogruppe ist.

3. Verbindungen nach Anspruch 1 oder 2, worin R eine Isopropyl-, 1-Methylpropyl-, Isobutyl-, Cyclopentyl- oder Cyclohexylgruppe ist.

4. Verbindungen nach Anspruch 1, worin R Isopropyl ist, $R^1$ Chlor ist, $R^2$ Wasserstoff ist und $R^3$ Wasserstoff ist.

5. Herbicide Zusammensetzungen, welche als aktiven Bestandteil eine in Anspruch 1 definierte Verbindung der Formel (I) in Mischung mit einem aus einem festen oder flüssigen Verdünnungsmittel bestehenden Träger enthalten.

6. Herbicide Zusammensetzungen, welche mindestens eine in Anspruch 1 definierte Verbindung der Formel (I) in Mischung mit mindestens einem weiteren nicht die Formel (I) aufweisenden Herbicid enthalten.

7. Verfahren zur Verhinderung des Wachstums von unerwünschten Pflanzen, bei welchem auf die Pflanzen oder auf deren Wachstumsort eine in Anspruch 1 definiert Verbindung der Formel (I) in einer herbicid wirksamen Menge aufgebracht wird.

8. Verfahren nach Anspruch 7, bei welchem die Aufbringrate der Verbindung der Formel (I) 0,5 bis 10 kg/ha beträgt.

9. Verfahren zur Herstellung der in Anspruch 1 definierten Verbindungen der Formel (I), wobei R, $R^1$ und $R^2$ der Definition von Anspruch 1 entsprechen und $R^3$ Wasserstoff ist, bei welchem ein Alkyl- oder Cycloalkyl-bis-chloroformylamin der Formel $RN(COCl)_2$ mit einem 3-amino-4,5-$R^1R^2$-disubstituierten Pyrazol umgesetzt wird und die Verbindung der Formel (I), worin $R^3$ Wasserstoff ist, abgetrennt wird.

10. Verfahren zur Herstellung von Verbindungen der Formel (I), wobei die Gruppe $R^1$ Brom, Chlor, Jod oder Nitro ist, bei welchem eine Verbindung der Formel (I), worin $R^1$ Wasserstoff ist, mit einem Halogenierungs- oder Nitrierungsmittel umgesetzt wird.